# FASCICULE DE BREVET EUROPEEN

(11) **EP 4 346 591 B1**
(45) Date de publication et mention de la délivrance du brevet: **04.02.2026**
(21) Numéro de dépôt: 22731753.4
(22) Date de dépôt: 25.05.2022
(51) Int. Cl.: A61B 5/145, G06F 1/16, H01M 50/216, H01M 50/258, H01M 50/262, H01M 50/269, H01M 50/296, A61B 5/00

(54) **SYSTÈME DE CHARGE D'UN DISPOSITIF ÉLECTRONIQUE DE SURVEILLANCE D'UN PARAMÈTRE PHYSIOLOGIQUE**
LADESYSTEM FÜR EIN ELEKTRONISCHES GERÄT, VORZUGSWEISE FÜR EIN ELEKTRONISCHES GERÄT ZUR ÜBERWACHUNG EINES PHYSIOLOGISCHEN PARAMETERS
CHARGING SYSTEM FOR AN ELECTRONIC DEVICE, PREFERABLY FOR AN ELECTRONIC DEVICE FOR MONITORING A PHYSIOLOGICAL PARAMETER

(30) Priorité: 27.05.2021 FR 2105513
(43) Date de publication de la demande: 10.04.2024
(73) Titulaire: WIZP AS, 3125 Tønsberg (NO)
(72) Inventeur: PIERART, Luc, 94800 Villejuif (FR); LE, Anh-minh, 92200 Neuilly sur Seine (FR)
(74) Mandataire: Regimbeau
(86) Numéro de dépôt international: PCT/FR2022/050994
(87) Numéro de publication internationale: WO 2022/248806

(56) Documents cités:
- FR-A1- 3 084 777
- US-A1- 2003 078 501
- US-A1- 2007 043 959
- US-A1- 2014 111 147
- CHANNEL WELL TECHNOLOGY: "Stackable Wireless Charging Power Bank Suite - Operating Manual", 19 July 2016 (2016-07-19), Taoyuan City 338, Taiwan, pages 1 - 3, XP093243417, Retrieved from the Internet <URL:http://www.channelwell.com/en/ImgShowroom/20160719165800.pdf>
- CHANNEL WELL TECHNOLOGY: "Stackable Wireless Charging Power Bank Suite - Products - Channelwell", 19 July 2016 (2016-07-19), pages 1 - 4, XP093243423, Retrieved from the Internet <URL:http://www.channelwell.com/en/Showroom/ugC_ShowroomItem_Detail.asp?hidshowid=26>
- CLOVER JULI: "WiBa Wireless and Stackable Power Bank and Charging Pad Review - MacRumors", 16 November 2018 (2018-11-16), Glen Allen, VA 23060, pages 1 - 14, XP055880126, Retrieved from the Internet <URL:https://www.macrumors.com/review/wiba-wireless-stackable-power-bank/> [retrieved on 20220117]

## Description

### DOMAINE TECHNIQUE

L'invention concerne un système de charge d'un dispositif électronique et de préférence d'un dispositif électronique de surveillance d'un paramètre physiologique d'un utilisateur, notamment via l'analyse d'un liquide corporel, typiquement interstitiel au moyen de microaiguilles. Un tel dispositif électronique peut aussi être une montre connectée ou un bracelet connecté. Plus précisément, l'invention vise à rendre possible une utilisation la plus continue de ces dispositifs.

### ETAT DE LA TECHNIQUE

Certaines pathologies comme le diabète nécessitent une surveillance quotidienne de paramètres biochimiques du corps humain, c'est-à-dire des concentrations en certains composés (la glycémie dans l'exemple du glucose).

On connait des dispositifs avec microaiguilles, qui ont l'avantage d'être moins invasive que des aiguilles classiques, comme celui décrit dans le document WO2018104647, qui présente un boitier comprenant une capsule amovible, la capsule accueillant des microaiguilles configurées pour prélever du liquide interstitiel. Le boitier, quant à lui, accueille la majeure partie de l'électronique.

Ce dispositif portatif, typiquement au poignet, permet une mesure en continue et il suffit de changer la capsule pour changer de microaiguilles.

Toutefois, ce dispositif portatif a besoin d'être rechargé.

Pour ce faire, une base de charge est prévue à cet effet, le dispositif électronique est alors connecté à la base qui elle-même est connectée à une source d'alimentation pour pouvoir charger le dispositif électronique.

Un problème est la mobilité. En effet, l'utilisateur doit toujours s'assurer de disposer d'une source de courant pour charger son dispositif ce qui en termes de mobilité n'est pas satisfaisant. Pour pallier ce problème l'utilisateur peut prévoir une batterie externe portable (en anglais, powerbank) afin de toujours disposer d'une source d'alimentation. Ceci n'est toujours pas satisfaisant car l'utilisateur doit prévoir de pouvoir charger la batterie externe et disposer de la connectique nécessaire pour brancher la batterie externe.

Le système de charge 2AAT010B commercialisé par la société Guangzhou Gui Guan Power Plus Tech Corp (documents XP093243417 et XP093243423) comprend une base de charge WCB005V et une batterie externe WCH003A. Et un système similaire nommée WiBa a également été commercialisé par la société Avido (document XP055880126).

Mais en termes d'expérience utilisateur et de mobilité il existe un besoin de proposer un système de charge simple à utiliser, fiable et qui améliore l'expérience utilisateur notamment en termes de mobilité et qui donc nécessite une connectique la plus simple possible.

### EXPOSE DE L'INVENTION

L'invention propose de pallier au moins un de ces inconvénients.

A cet effet, l'invention propose un ensemble selon la revendication indépendante 1.

L'invention est avantageusement complétée par les caractéristiques suivantes, prises seules ou en une quelconque de leur combinaison techniquement possible :
- la batterie externe et la base comprennent des connecteurs électriques complémentaires de manière à ce que la batterie externe et la base soient en contact électrique lorsqu'elles sont assemblées ;
- les moyens de solidarisation de la base à la batterie externe comprennent des aimants s'attirant l'un vers l'autre lorsqu'ils sont à proximité ;
- les moyens de solidarisation de la base au dispositif électronique comprennent des aimants s'attirant les uns vers les autres lorsqu'ils sont à proximité ;
- les moyens de solidarisation de la base à la batterie externe comprennent des éléments de clipsage, pour que la face de la batterie épouse la base en contact ;
- la base et la batterie externe comprennent des formes complémentaires afin de faciliter la solidarisation de la base avec la batterie.

Les avantages de l'invention sont multiples.

L'invention permet d'étendre l'utilisation du dispositif électronique sans accès à l'électricité afin d'avoir à disposition les informations vitales fournies par le dispositif ce qui peut être nécessaire dans des situations critiques (trek prolongé, bateau en panne, etc.). L'utilisateur est donc plus en sécurité dans ces cas d'utilisations nomades.

L'invention fournit un système de charge compact et mobile qui correspond à ce besoin.

Le fait de pouvoir détacher la batterie externe à la demande de la base permet de faciliter son remplacement. En effet, si la batterie externe ne fonctionne plus il est possible de la remplacer indépendamment de la base. De même si la base ne fonctionne plus, il est possible de la remplacer indépendamment de la batterie externe.

En termes de mobilité, il est en outre possible de prévoir plusieurs batteries externes si l'utilisateur n'a pas la possibilité de recharger la batterie externe ou son dispositif électronique via une alimentation électrique conventionnelle.

### PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :
- la figure 1 illustre un système de charge selon l'invention ;
- la figure 2 illustre un dispositif électronique de surveillance d'un paramètre physiologique connecté au système de charge selon l'invention ;
- la figure 3 illustre une batterie portable de charge d'un système de charge selon l'invention ;
- la figure 4 illustre une base en perspective vue de dessus d'un système de charge selon l'invention ;
- la figure 5 illustre une batterie portable de charge en perspective vue du dessous d'un système de charge selon l'invention ;
- la figure 6 illustre une batterie portable de charge vue de dessous d'un système de charge selon l'invention ;
- la figure 7 illustre un dispositif électronique connecté à une base d'un système de charge selon l'invention.

Sur l'ensemble des figures les éléments similaires portent des références identiques.

### DESCRIPTION DETAILLEE

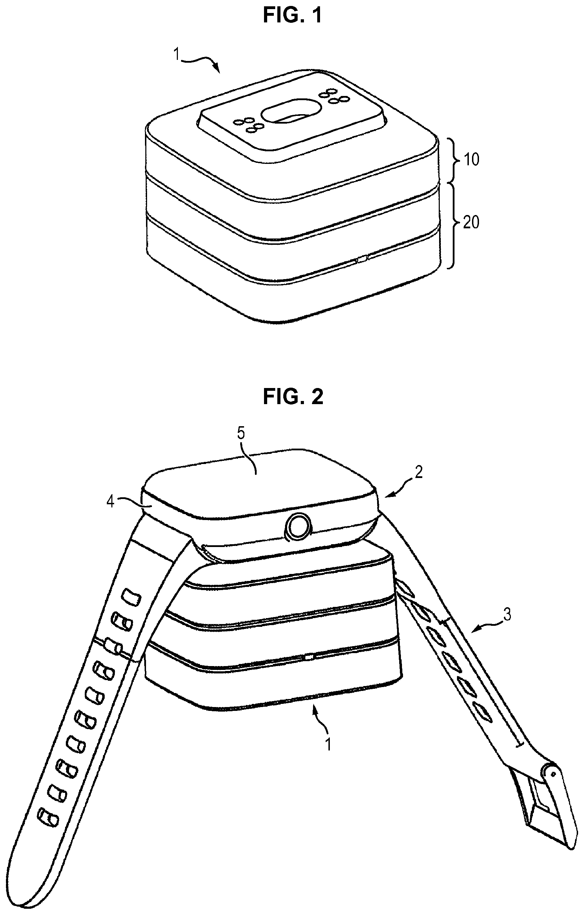

La **figure 1** illustre un système de charge 1 d'un dispositif électronique de surveillance d'un paramètre physiologique d'un individu. Le système de charge 1 comprend une base support de charge 10 (en anglais dock) et une batterie portable 20 externe rechargeable (en anglais powerbank). Ci-après on désignera par batterie portable de charge la batterie portable 20 externe rechargeable et par batterie interne, la batterie interne du dispositif électronique 2. Comme cela sera détaillé plus loin, cette batterie portable de charge 20 n'est utilisée que pour charger la batterie interne du dispositif électronique 2. Il ne s'agit donc pas d'une batterie supplémentaire qui remplace la batterie interne du dispositif électronique 2 lorsque celle-ci est déchargée.

La **figure 2** illustre un dispositif électronique 2 de surveillance d'un paramètre physiologique d'un individu disposé sur le système de charge 1. Un tel dispositif électronique 2 se présente ici sous la forme d'une montre qui peut être portée à un membre de l'individu (bras, avant-bras, jambe, cuisse, mollet, etc.).

Le dispositif électronique 2 comprend un bracelet 3 ou une lanière lui permettant de s'attacher à un membre et un boitier 4. Le boitier 4 comprend un écran d'affichage 5.

Le dispositif électronique 2 est de préférence un dispositif qui comprend des microaiguilles configurées pour être insérées dans la peau (dans une partie superficielle de l'épiderme). Ces microaiguilles permettent de prélever et/ou d'analyser un fluide corporel.

Dans le boitier 4 sont disposés des moyens de traitement de données (en particulier un processeur ou un microcontrôleur) configurés pour traiter des mesures issues des microaiguilles.

Le dispositif électronique 2 se connecte à la base 10 afin d'être chargé par son intermédiaire.

S'agissant d'un dispositif électronique 2 celui-ci comprend une batterie interne logée dans le boitier 4 (non illustrée). Dans ce qui suit on parlera de la charge du dispositif électronique pour désigner la charge de sa batterie interne.

Comme présenté en introduction, un tel dispositif électronique 2 nécessite d'être chargé. En particulier il est chargé au moyen du système de charge 1.

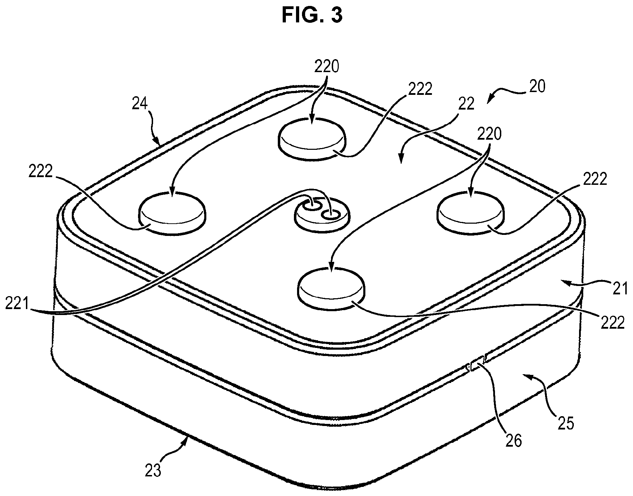

La batterie portable de charge 20 illustrée sur la **figure 3** comprend un corps polygonal 21 qui comprend plusieurs faces 22, 23, 24, 25.

De préférence, il s'agit d'un parallélépipède comprenant une longueur et une largeur. L'épaisseur est adaptée à la capacité désirée de la batterie. Sur la **figure 3** la batterie portable de charge 20 a une forme parallélépipédique avec des angles arrondis mais elle peut aussi être oblongue.

Un accumulateur est logé l'intérieur du corps 21. L'accumulateur permet de stocker et restituer de l'énergie. On précise que la batterie portable de charge 20 est à charge rapide compte tenu que l'utilisateur doit pouvoir se passer du dispositif électronique 2 seulement pendant une durée déterminée qui ne doit pas être trop longue. Une telle durée est d'environ trente minutes dans le cas d'un dispositif électronique 2 pour mesurer le taux de glucose. Ainsi, une charge en vingt-trente minutes est acceptable sans que cela puisse porter préjudice à l'utilisateur qui doit pouvoir utiliser le dispositif électronique 2 avec des interruptions qui soient les plus courtes possibles.

Un indicateur visuel 26 tel qu'une diode peut être prévu sur une des faces afin d'informer de l'état de charge de la batterie portable de charge 20.

La batterie portable de charge 20 peut comprendre sur une de ses faces, ici la face supérieure 22 des contacts électriques 221, par exemple, deux contacts électriques situés de préférence au centre de la face 221. Ces contacts électriques 221 permettent de connecter électriquement la batterie portable de charge 20 à la base 10. Dans le contexte de l'invention, pour permettre le transfert d'énergie de la batterie portable de charge 20 vers la base 10 on prévoit une zone inductive sur la batterie portable de charge 20 pour, par induction, transférer de l'énergie depuis la batterie portable de charge 20 vers la base 10.

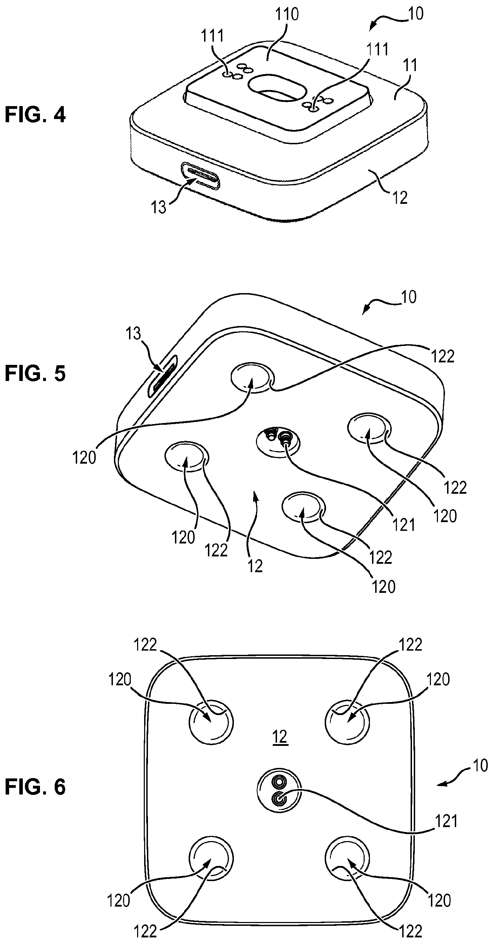

Comme illustré sur les **figures 4, 5 et 6****,** la base 10 comprend une partie supérieure 11 et une partie inférieure 12.

La partie inférieure 12 de la base 10 comprend des contacts électriques 121 complémentaires à ceux présents sur la batterie portable de charge 20 afin de connecter électriquement la base 10 à la batterie portable de charge 20. Ces contacts électriques 121 sont situés au centre la partie inférieure 12. De manière alternative, pour permettre le transfert d'énergie de la batterie portable de charge 20 vers la base 10 on peut prévoir une zone inductive sur cette partie inférieure pour, par induction, transférer de l'énergie depuis la batterie portable de charge 20 vers la base 10.

Afin de connecter le dispositif électronique 2 à la partie supérieure 11 de la base 10, des contacts électriques 111 peuvent être prévus au centre de cette partie supérieure.

La partie supérieure 11 de la base comprend des moyens de solidarisation 110 de manière amovible à la demande du dispositif électronique 2 de manière à solidariser et désolidariser le dispositif électronique 2 de la base 10 à la demande. La solidarisation est dite amovible à la demande le dispositif électronique 2 doit pouvoir être connecté et déconnecté à volonté.

Ces moyens de solidarisation comprennent des aimants qui sont de préférence disposés sur une zone 110 en saillie de la base 10 afin de faciliter la connexion du dispositif 2 à la base 10, le dispositif 2 comprenant une forme complémentaire à cette zone 110.

La partie inférieure 12 de la base 10 comprend des moyens de solidarisation 120 de manière amovible d'une face de la batterie portable de charge 20 de manière à solidariser et désolidariser la batterie de la base support à la demande.

Ainsi, la face supérieure 22 de la batterie portable de charge 20 comprend aussi des moyens de solidarisation 220 de manière amovible à la demande de la batterie portable de charge 20 à la base 10 de manière à solidariser et désolidariser la batterie portable de charge 20 de la base 10 à la demande. Ces moyens de solidarisation 110, 220 autorisent une liaison complète sans degré de liberté entre la base 10 et la batterie portable de charge 20.

Ici encore, la solidarisation est dite amovible à la demande car la batterie et la base doivent pouvoir se connecter et se déconnecter à volonté.

Les moyens de solidarisation de la base à la batterie portable de charge 20 sont donc complémentaires.

L'idée est de connecter directement la base 10 à la batterie portable de charge 20 sans cordon d'alimentation dédié afin de pouvoir alimenter la base 10 au moyen de la batterie portable de charge 20 portable afin qu'elle puisse charger le dispositif électronique 2 ou afin qu'elle puisse charger la batterie portable de charge 20. A ce titre, la batterie portable de charge 20 ne comprend pas de connecteur dédié au branchement d'un quelconque cordon comme c'est très souvent le cas avec les batteries portables ou powerbank classiques.

Les moyens de solidarisation de la base à la batterie portable de charge 20 peuvent prendre plusieurs formes. Il peut s'agir d'une solidarisation magnétique ou par clipsage.

Selon un mode de réalisation, ces moyens de solidarisation 120 comprennent des aimants positionnés dans la partie inférieure de la base 10, la batterie portable de charge 20 comprenant sur la face qui doit en contact avec la base des aimants complémentaires 220. Il suffit alors d'approcher la batterie portable 20 de charge de la base pour qu'il y ait solidarisation. De manière complémentaire, une complémentarité de forme peut être prévue afin de faciliter la solidarisation de la base à la batterie portable de charge 20. Ceci est visible sur les **figures 3** et **4** ou les moyens de solidarisation 120, 220 sont positionnés du côté de la batterie portable de charge 20 sur des dômes 222 en saille de la face qui doit être connectée à la base 10 et dans des cavités 122 sur la partie inférieure de la base 10. Bien sûr on peut prévoir que les moyens de solidarisation soient positionnés indépendamment des formes complémentaires facilitant la solidarisation.

Selon un mode de réalisation, des moyens de de solidarisation comprennent des pattes de clipsage qui sont positionnées sur la base de préférence. Les pattes ont une forme de C ou en angle droit. De manière plus générale, elles ont une forme qui permettent d'épouser la face de la batterie portable de charge 20 qui doit être clipsée à la base.

Selon un mode de réalisation, les moyens de solidarisation sont la combinaison des aimants complémentaires et des pattes de clipsage.

La base 10 comporte également un connecteur 13 permettant de relier la base à une source externe d'alimentation. Il peut s'agit par exemple d'un connecteur USB ou USB-C auquel est branché un cordon d'alimentation lui-même branché à une source d'alimentation (secteur ou powerbank). Il n'est pas nécessaire d'avoir à disposition un câble de connexion pour alimenter la base à partir de la batterie portable 20 de charge de l'invention.

La base 10 peut donc être alimentée soit par la batterie portable de charge 20 soit par une source externe.

Le système de charge 1 permet plusieurs cas d'utilisations.

Lorsque la base 10 est connectée à une source externe elle peut charger le dispositif électronique et/ou la batterie portable de charge 20. En effet, le dispositif électronique 2 et la batterie portable de charge 20 peuvent être connectés et chargés individuellement, comme cela est visible sur la **figure 2****.** Le dispositif électronique 2 et la batterie portable de charge 20 sont ainsi connectés simultanément à la base 10 pour pouvoir chacun être chargés. Ils sont ainsi chargés ensemble du point de vue de l'utilisateur.

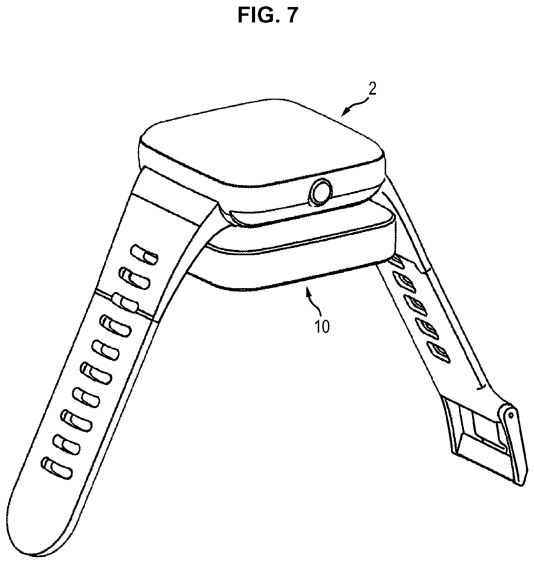

Lorsque la base 10 n'est pas connectée à une source externe avec la batterie portable de charge 20 connectée à sa face inférieure, alors la base sert à charger le dispositif électronique 2 à partir de la batterie portable de charge 20. Bien sûr, le dispositif électronique 2 peut être utilisé seul avec la base 10 comme cela est visible sur la **figure 7****.**

Lorsque la batterie portable de charge 20 rechargeable est solidaire de la base, la base 10 et la batterie portable de charge 20 forment un ensemble unitaire, visible sur la **figure 1****,** qui si on ne connecte pas la base 10 à une source externe ne nécessite pas de connecteur puisque tous les contacts sont directs entre les différents éléments.

## Revendications

1. Ensemble comprenant un dispositif électronique (2) de surveillance d'un paramètre physiologique d'un individu comprenant une batterie interne et un système de charge (1) de la batterie interne du dispositif électronique (2), le système de charge comprenant :
- une base (10) de charge du dispositif électronique (2) ayant une partie supérieure et une partie inférieure, la base (10) comprenant un connecteur (13) d'alimentation adapté pour relier la base (10) à une source externe d'alimentation (10) apte à alimenter la base (10) ;
- une batterie portable de charge (20) rechargeable comprenant plusieurs faces ; la partie supérieure (11) de la base de charge comprenant des moyens de solidarisation amovible du dispositif électronique ;
la partie inférieure (12) de la base de charge et une face de la batterie portable de charge (20) comprenant des moyens de solidarisation amovible de manière à solidariser et désolidariser la batterie portable de charge (20) de la base (10) de charge à la demande, le contact de la base avec la face de la batterie portable de charge (20) permettant :
- lorsque la base (10) est connectée à une source externe et est alimentée de charger le dispositif électronique et/ou de charger la batterie portable de charge (20) ;
- lorsque la base (10) n'est pas connectée à une source externe et n'est pas alimentée de charger le dispositif électronique (2) à partir de la batterie portable de charge (20) ;
dans lequel la batterie portable de charge (20) et la base (10) sont configurées pour être connectées par l'intermédiaire d'une zone de charge par induction, la batterie pouvant alimenter par induction la base (10).

2. Ensemble selon la revendication 1, dans lequel la batterie portable de charge (20) et la base (10) comprennent des connecteurs électriques (111, 221) complémentaires de manière à ce que la batterie portable de charge (20) et la base (10) soient en contact électrique lorsqu'elles sont assemblées.

3. Ensemble selon l'une des revendications précédentes, dans lequel les moyens de solidarisation de la base (10) à la batterie portable de charge (20) comprennent des aimants s'attirant l'un vers l'autre lorsqu'ils sont à proximité.

4. Ensemble selon l'une des revendications précédentes, dans lequel les moyens de solidarisation de la base (10) au dispositif électronique (2) comprennent des aimants s'attirant les uns vers les autres lorsqu'ils sont à proximité.

5. Ensemble selon l'une des revendications 1 et 2, dans lequel les moyens de solidarisation de la base à la batterie comprennent des éléments de clipsage, pour que la face de la batterie portable de charge (20) épouse la base (10) en contact.

6. Ensemble selon l'une des revendications précédentes, dans lequel la base (10) et la batterie comprennent des formes complémentaires afin de faciliter la solidarisation de la base avec la batterie portable de charge (20).

## Patentansprüche

1. Anordnung, umfassend eine elektronische Vorrichtung (2) zur Überwachung eines physiologischen Parameters eines Individuums, umfassend eine innere Batterie und ein Ladesystem (1) der inneren Batterie der elektronischen Vorrichtung (2), wobei das Ladesystem umfasst:
- eine Ladestation (10) für die elektronische Vorrichtung (2) mit einem oberen Teil und einem unteren Teil, wobei die Station (10) einen Versorgungsverbinder (13) umfasst, der zur Verbindung der Station (10) mit einer externen Versorgungsquelle (10) geeignet ist, die imstande ist, die Station (10) zu versorgen;
- eine wiederaufladbare tragbare Ladebatterie (20), die mehrere Flächen umfasst; wobei der obere Teil (11) der Ladestation Mittel zum lösbaren Befestigen der elektronischen Vorrichtung umfasst;
wobei der untere Teil (12) der Ladestation und eine Fläche der tragbaren Ladebatterie (20) Mittel zum lösbaren Befestigen umfassen, um die tragbare Ladebatterie (20) bei Bedarf an der Ladestation (10) zu befestigen und von dieser zu lösen, wobei der Kontakt mit der Fläche der tragbaren Ladebatterie (20) gestattet:
- wenn die Station (10) mit einer externen Quelle verbunden ist und versorgt wird, die elektronische Vorrichtung zu laden und/oder die tragbare Ladebatterie (20) zu laden;
- wenn die Station (10) nicht mit einer externen Quelle verbunden ist und nicht versorgt wird, die elektronische Vorrichtung (2) von der der tragbaren Ladebatterie (20) aus zu laden;
wobei die tragbare Ladebatterie (20) und die Station (10) ausgelegt sind, um über einen Ladebereich durch Induktion verbunden zu sein, wobei die Batterie die Station (10) durch Induktion laden kann.

2. Anordnung nach Anspruch 1, wobei die tragbare Ladebatterie (20) und die Station (10) komplementäre elektrische Verbinder (111, 221) umfassen, so dass die tragbare Ladebatterie (20) und die Station (10) in elektrischem Kontakt stehen, wenn sie miteinander verbunden sind.

3. Anordnung nach einem der vorstehenden Ansprüche, wobei die Mittel zum Befestigen der Station (10) an der tragbaren Ladebatterie (20) Magnete umfassen, die sich gegenseitig anziehen, wenn sie sich in der Nähe befinden.

4. Anordnung nach einem der vorstehenden Ansprüche, wobei die Mittel zum Befestigen der Station (10) an der elektronischen Vorrichtung (2) Magnete umfassen, die sich gegenseitig anziehen, wenn sie sich in der Nähe befinden.

5. Anordnung nach einem der Ansprüche 1 und 2, wobei die Mittel zum Befestigen der Station an der Batterie Clip-Elemente umfassen, damit die Fläche der tragbaren Ladebatterie (20) in Kontakt mit der Station (10) steht.

6. Anordnung nach einem der vorstehenden Ansprüche, wobei die Station (10) und die Batterie komplementäre Formen aufweisen, um die Befestigung der Station an der tragbaren Ladebatterie (20) zu erleichtern.

## Claims

1. An assembly comprising an electronic device (2) for monitoring a physiological parameter of an individual comprising an internal battery and a charging system (1) for the internal battery of the electronic device (2), the charging system comprising:
- a charging base (10) for the electronic device (2) having an upper part and a lower part, the base (10) comprising a power connector (13) adapted to connect the base (10) to an external power source (10) capable of supplying power to the base (10);
- a portable rechargeable charging battery (20) comprising several sides; the upper part (11) of the charging base comprising means for removably securing the electronic device;
the lower part (12) of the charging base and one side of the portable charging battery (20) comprising removable fastening means for fastening and unfastening the portable charging battery (20) to and from the charging base (10) as required, ing contact between the base and the side of the portable charging battery (20) allowing:
- when the base (10) is connected to an external source and is powered, to charge the electronic device and/or to charge the portable charging battery (20);
- when the base (10) is not connected to an external source and is not powered, to charge the electronic device (2) from the portable charging battery (20);
wherein the portable charging battery (20) and the base (10) are configured to be connected via an induction charging area, the battery being able to supply power to the base (10) by induction.

2. An assembly according to claim 1, wherein the portable charging battery (20) and the base (10) comprise complementary electrical connectors (111, 221) such that the portable charging battery (20) and the base (10) are in electrical contact when assembled.

3. Assembly according to one of the preceding claims, wherein the means for securing the base (10) to the portable charging battery (20) comprise magnets that attract each other when they are close together.

4. Assembly according to one of the preceding claims, wherein the means for securing the base (10) to the electronic device (2) comprise magnets that attract each other when they are close together.

5. Assembly according to one of claims 1 and 2, wherein the means for securing the base to the battery comprise clip elements, so that the face of the portable charging battery (20) fits snugly against the base (10).

6. Assembly according to one of the preceding claims, wherein the base (10) and the battery comprise complementary shapes in order to facilitate attachment of the base to the portable charging battery (20).
